# FASCICULE DE BREVET EUROPEEN

(11) **EP 3 938 163 B1**
(45) Date de publication et mention de la délivrance du brevet: **29.10.2025**
(21) Numéro de dépôt: 20709617.3
(22) Date de dépôt: 13.03.2020
(51) Int. Cl.: B29B 17/02, B29B 17/04, B29L 31/00, B29K 105/10

(54) **PROCÉDÉ POUR RECYCLER DES CORDAGES**
VERFAHREN ZUM RECYCLING VON SEILEN
METHOD FOR RECYCLING ROPES

(30) Priorité: 13.03.2019 FR 1902553
(43) Date de publication de la demande: 19.01.2022
(73) Titulaire: Menneglier, Jean-Mathieu, 78750 Mareil-Marly (FR)
(72) Inventeur: Menneglier, Jean-Mathieu, 78750 Mareil-Marly (FR)
(74) Mandataire: Cabinet Didier Martin
(86) Numéro de dépôt international: PCT/EP2020/056985
(87) Numéro de publication internationale: WO 2020/183018

(56) Documents cités:
- BE-A- 445 393
- CN-A- 107 738 382
- DE-A1- 102013 200 482
- DE-A1- 19 507 513
- FR-A1- 3 007 412
- KORZEN Z: "RECOVERY OF RUBBER AND STEEL CORDS FROM CONVEYOR BELTS", INTERNATIONAL POLYMER SCIENCE AND TECHNOLOGY, RAPRA TECHNOLOGY, SHREWABURY, GB, vol. 19, no. 12, 1 January 1992 (1992-01-01), pages T51 - T57, XP000363234, ISSN: 0307-174X

## Description

### Domaine technique

L'invention se rapporte aux procédés et dispositifs pour recycler des cordages. On s'intéresse en particulier au recyclage des cordages de raquettes de sport. Toutefois l'invention vise aussi le recyclage de cordages techniques tels que ceux utilisés pour la voile, la varappe, l'alpinisme ou encore d'autres sports. Il est également considéré de pouvoir appliquer la présente invention aux cordages d'instruments de musique notamment des instruments à cordes.

### Contexte et Art antérieur

Actuellement, les cordages cassés ou usagés des raquettes de sport (tennis, squash, badminton) sont mis à la poubelle ou jetés sans précaution.

Tant que, dans une situation antérieure, les cordages étaient obtenus à partir de fibres naturelles d'origine animale ou végétale, ils pouvaient être considérés comme des matériaux naturellement bio-dégradables et leur mise en décharge voire leur rejet dans un endroit quelconque de la nature ne posait pas de problème sous l'angle environnemental.

Or il s'avère que dans des cas de plus en plus nombreux, ces cordes comprennent des composants synthétiques et des fibres techniques qui ne sont pas biodégradables directement. Leur mise à la poubelle entraîne donc un impact défavorable du point de vue écologique sur l'environnement. Certains composants peuvent être considérés comme des déchets toxiques.

On constate une pression des pouvoirs publics et des organisations non-gouvernementales pour gérer les déchets liés à l'activité humaine et parvenir à établir réellement une économie circulaire et des taux de recyclage très élevés.

Les documents CN 107 738 382 A, FR 3 007 412 A1 et BE 445 393 A décrivent des procédés de recyclage de l'art antérieur.

La présente invention propose une solution selon laquelle beaucoup de cordages pourront être recyclés.

### Résumé de l'invention

A cet effet, il est proposé un procédé pour recycler des cordages 30 composites, le procédé comprenant:
- une étape d'identification du type de cordage,

et au moins une étape de séparation de composants comprenant l'une ou l'autre des étapes suivantes /S1 /,/S2/,/S3/,/S4/ telles que définies ci-après :
   /S1/ - une étape de séparation mécanique,
   /S2/ - une étape de séparation chimique,
   /S3/ - une étape de séparation thermique,
   /S4/ - une étape de séparation biologique,
procédé dans lequel les cordages à recycler sont des cordages techniques utilisés pour des raquettes de tennis, pour la voile, la varappe, l'alpinisme ou encore d'autres sports,
ou des cordages d'instruments de musique à cordes, et dans lequel :
   - l'étape d'identification (SO) pour les cordages de raquettes de tennis consiste à pratiquer une coupe transversale, prendre une photo avec grossissement, et analyser les groupes de filaments, pour en déduire une classification parmi au moins les types suivants : multi filaments simple, multi-filaments multi-cœur, multi-cœur multi-gaines, multi-cœur mono-gaine.

Grâce à ces dispositions, on peut recycler les cordages notamment les cordages de raquettes de sport, et éviter ainsi de les jeter à la poubelle.

Ce procédé permet de mettre en place une « économie circulaire verte » autour du traitement des cordages, notamment des cordages de raquettes. L'économie circulaire va permettre d'utiliser le déchet polluant en transformant sa composition en une ressource valorisable.

Selon sa composition, on reconvertit chacune des composantes du cordage en la réintroduisant dans un nouveau cycle de production.

Il est ainsi proposé une solution permettant de ne pas jeter son cordages dans les ordures ménagères résiduelles, afin que ceux-ci ne soient non plus éliminés, mais valorisés ; grâces à des solutions techniques de recyclage de proximité ou de collecte valorisée.

Selon l'invention, l'étape d'identification pour les cordages, tels que les cordages de raquettes de tennis, consiste à pratiquer une coupe transversale, prendre une photo avec grossissement, et analyser les groupes de filaments, pour en déduire une classification parmi au moins les types suivants : multi filaments simples, multi filaments multi cœur, multi cœur multi gaine, multi cœur mono gaine. Moyennant quoi, on peut choisir ensuite, en fonction de l'identification obtenue, la ou les étapes de séparation les plus appropriées.

Dans divers modes de réalisation de l'invention concernant le système, on peut éventuellement avoir recours en outre à l'une et/ou à l'autre des dispositions suivantes, prises isolément ou en combinaison.

Selon une option d'intérêt, les cordages à recycler sont des cordages de raquettes de sport avec cordes. On englobe dans les sports avec cordes notamment mais non exclusivement: le tennis, le badminton, le squash.

Selon une autre option, le procédé proposé permet les cordages techniques utilisés pour la voile, la varappe, l'alpinisme ou encore d'autres sports. Selon une autre option, le procédé proposé permet les cordages d'instruments de musique notamment des instruments à cordes comme violon, violoncelle, guitare, contrebasse.

Selon une option d'intérêt, les cordages à recycler sont des cordages de raquettes de tennis. Le présent inventeur a découvert que pour de cordages de raquettes de tennis, les quantités de cordages composites étaient de plus en plus importantes. D'autre part, tant pour les joueurs professionnels que pour les joueurs amateurs aguerris, lesdits joueurs ont tendance à procéder au remplacement du cordage avant que celui ne casse ou n'atteigne sa réelle fin de vie, ce qui augmentent considérablement les quantités consommées et renforce l'intérêt de la solution de recyclage proposé.

Selon une option, les cordages de raquettes de tennis comprennent un assemblage de fibres de structure avec un matériau d'enrobage, les fibres de structure comprenant des fibres d'aramide et/ou de polyester et/ou de polyamide et/ou de polyoléfine et/ou de polyéthylène, et le matériau d'enrobage comprenant du polyuréthane et/ou un élastomère. Le procédé proposé permet ainsi de traiter une très grande variété de types de cordages technique pour le tennis ou des cordages pour d'autres usages. La plupart des composants usuels en terme de fibres sont ainsi pris en compte et on peut atteindre un taux de recyclabilité très élevé.

Selon une option, pour l'étape de séparation mécanique (S1) des fibres et de l'enrobage, il est prévu une étape de pelage et/ou une étape de broyage du fil de cordage. Moyennant quoi, lorsque la longueur des cordes à traiter le permet, la corde peut être passer dans une machine similaire à une extrudeuse dans laquelle des râpes viennent peler le matériau d'enrobage au moins pour sa partie périphérique. Quant à l'étape de broyage, cette dernière permet de découper le fil de cordage en petit morceaux qui peuvent être traités ensuite par une étape de séparation chimique, thermique ou biologique.

Selon une option, pour l'étape de séparation chimique il peut être prévu une dissolution de l'enrobage des fibres dans un solvant. Moyennant quoi, il est possible de séparer de manière complète et fiable les fibres techniques du matériau d'enrobage.

Selon une option, l'étape de séparation thermique peut comprendre une étape de vapo-thermolyse, avec un réchauffement du fil de cordage au-delà de 150°. Moyennant quoi, en fonction des points de fusions des différents composants, on peut séparer progressivement en augmentant la température et récupérant d'abord les composants qui ont le point de fusion de fusion le plus bas et en dernier les composants qui ont le point de fusion de fusion le plus haut.

Selon une option complémentaire, l'étape de séparation biologique comprend la mise en contact prolongée des fils de cordage avec des agents biologiques actifs comprenant des micro-algues et/ou des enzymes, de sorte que certains composants d'intérêt des fils de cordage sont dégradés par lesdits agents biologiques actifs. Moyennant quoi, même si le temps nécessaire est plus long, cette solution est la plus économique en énergie pour parvenir à la séparation des composants.

Selon une option complémentaire, on peut utiliser une telle étape de séparation biologique pour traiter par recyclage les cordages en boyau naturel. Les agents biologiques actifs comprenant des micro-algues et/ou des enzymes dégradent les cordages en boyau naturel en composants chimiques élémentaires qui n'ont alors plus aucun impact problématique sur l'environnement.

Selon une option on peut comparer la coupe transversale obtenue avec des coupes de référence, par exemple au moins d'une application Smartphone^{®}.

La présente invention vise aussi une machine pour recycler des cordages composites, notamment des cordages de raquettes de tennis, caractérisé en ce qu'elle est configurée pour mettre en œuvre, en partie ou en totalité, le procédé décrit ci-dessus.

### Brève description des dessins

D'autres aspects, buts et avantages de l'invention apparaîtront à la lecture de la description suivante d'un mode de réalisation de l'invention, donné à titre d'exemple non limitatif. L'invention sera également mieux comprise en regard des dessins joints sur lesquels:
- La figure 1 représente une illustration d'une raquette de tennis avec son cordage,
- La figure 2 illustre plusieurs types de section de cordage à recycler,
- La figure 3 représente une étape de séparation mécanique par pelage,
- La figure 4 illustre une étape de séparation mécanique par broyage,
- La figure 5 illustre une étape de séparation chimique,
- La figure 6 illustre une étape de séparation thermique,
- La figure 7 illustre une étape de séparation biologique.

### Description des modes de réalisation

Sur les différentes figures, les mêmes références désignent des éléments identiques ou similaires. Pour des raisons de clarté de l'exposé, certains éléments ne sont pas nécessairement représentés à l'échelle.

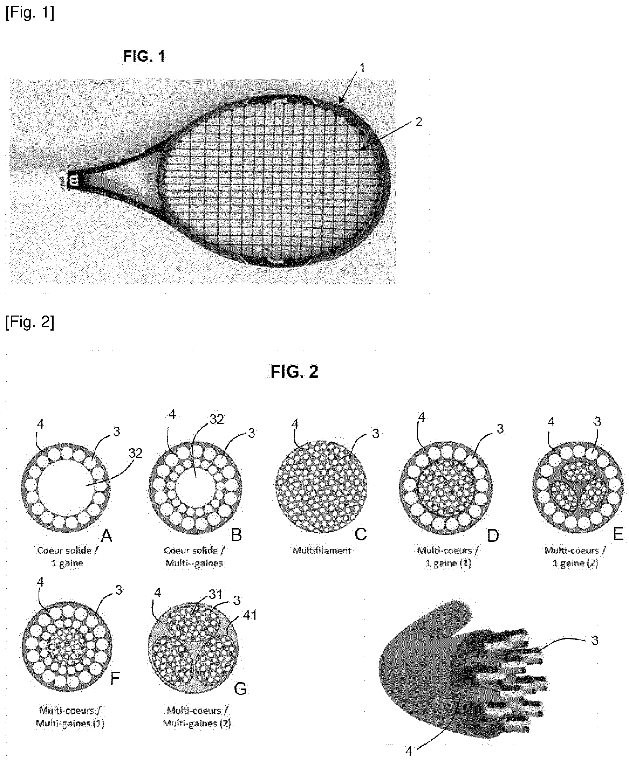

La figure 1 représente partiellement une raquette de tennis avec son cordage. La raquette comprend un cadre 1 et un cordage 2. Le cordage passe au travers de trous ménagés dans le cadre. Le cordage forme un tamis. Des sections longitudinales croisent des sections transverses. Pour terminer l'opération d'installation du cordage, on pratique un ou deux nœuds.

Le cordage 2 peut se rompre au bout d'une certaine usure ou bien si la contrainte endurée excède le seuil tolérable par le type de cordage en question. Certains utilisateurs ou joueurs remplacent le cordage de manière préventive comme déjà mentionné.

Pour ôter le cordage du cadre, on peut retirer les fils de cordage au travers des trous du cadre, après avoir pratiqué une ou plusieurs sectionnements du cordage.

On s'intéresse au cas très fréquent où le cordage 2 lui-même est formé un assemblage de fibres de structure avec un matériau d'enrobage. Quand on parle plus en détails de la composition du cordage, on peut aussi employer le terme « fil de cordage » pour désigne un élément unitaire de l'ensemble du cordage. Le fil de cordage peut dans certains cas comprendre un cœur solide 32.

On s'intéresse en particulier aux fils de cordage ayant un diamètre extérieur compris entre 0,8mm et 1,6 mm. Selon l'exemple, on traite en particulier des fils de cordage ayant un diamètre extérieur compris entre 1 mm et 1,5 mm

En ce qui concerne les fils de corde pour les raquettes de tennis, la section transversale du fil présente un diamètre compris entre 1,2 mm et 1,4 mm. Toutefois, en note que des diamètres plus petits aux plus grands sont également pris en compte dans le procédé de la présente invention.

Le matériau d'enrobage, repéré 4, comprend du polyuréthane et/ou un élastomère. On note que pour désigner I'« enrobage», on parle aussi de « gainage». Le matériau d'enrobage présente des propriétés mécaniques, s'agissant de la résistance en traction, inférieure aux fibres de structures ; mais le matériau d'enrobage participe à la cohésion du fil de cordage.

Comme illustré à la Figure 2, la structure du fil de cordage peut être de plusieurs types : multi filaments simples, multi filaments multi cœur, multi cœur multi gaine, multi cœur mono gaine.
Figure 2, exemple A : cœur solide, 1 gaine
Figure 2, exemple B : cœur solide, multi gaines
Figure 2, exemple C : multi filaments, sans coeur
Figure 2, exemple D : multi cœur solide, 1 gaine
Figure 2, exemple E : multi cœur solide, 1 gaine
Figure 2, exemple F : multi cœur, multi gaines
Figure 2, exemple G : multi cœur, multi gaines

Les fibres de structure, notées généralement 3 aux figures, comprennent des fibres d'aramide et/ou de polyester et/ou de polyamide et/ou de polyoléfine et/ou de polyéthylène.

Les fibres de structure utilisés pour les cordages de tennis ou pour d'autres types de cordage (cf liste plus haut) comprennent notamment :
- Aramide: Zylon (PBO) ; Kevlar (PPD-T) ; Kevlar 49 ; Black Technora,
- Polyester: Pen ou Pentex (PEN) ; Polyester (PES),
- Polyamide : Nylon ; Polyamide,
- Polyoléfines: Spectra ou Dyneema; Polyethylene PEHD,
- Titane : Titanium (Ti),
- Carbone : Fibres Carbone,
- Elastomère : Polybutylène ; élastomères (caoutchoucs).

### ARAMIDES

Les fibres d'aramide sont reconnues pour leur excellente résistance aux impacts, et sont très largement utilisées dans la fabrication d'équipements individuels de protection (casques, gants anti-coupures, gilets pare-balles, etc.). Dans le nautisme, les produits Kevlar^{®} ou Technora^{®} sont appréciés pour leur très bonne résistance et leur faible allongement à la rupture (environ 3,5%), ainsi que leur remarquable stabilité sous charge statique (pas de fluage). Autrement dit, ces fibres sont très solides (cinq fois plus que l'inox), très faiblement élastiques et ne s'allongent pas dans le temps.

Les fibres d'aramides sont aussi utilisées en tant que renforts de matériaux composites, afin d'offrir une bonne stabilité en température aux produits finaux (jusqu'à 200°C en fonction de la matrice). On peut toutefois noter quelques points moins positifs : résistance limitée aux UV et coût non négligeable.

### POLYESTERS

Les fibres de polyester sont des fibres de grande consommation, reconnues pour leur longévité, leur résistance aux UV et leur excellente résistance mécanique et chimique.

La fibre de polyester peut être recyclée de manière mécanique ou chimique, chacune représentant certains avantages et inconvénients. La méthode mécanique consiste à récupérer des bouteilles de plastique et des déchets industriels, tandis que le procédé chimique réutilise des produits textiles faits de polyester afin de les réduire en monomères puis de les retransformer en textiles.

La méthode de recyclage du polyester permet de recréer des matières quasi indéfiniment : les articles ou tissus pourront être recyclés de nombreuses fois sans perdre en qualité. De plus, le polyester recyclé produit par voie chimique ne comporte pas de métaux lourds, contrairement à son homologue fait avec du pétrole fraichement extrait.

### POLYAMIDES

La fibre de polyamide, ou nylon, est une fibre synthétique, dite « technique ». Elle est utilisée dans les applications industrielles textiles et plastiques, et trouve une application dans une large gamme de produits nécessitant des matériaux de haute résistance. Le polyamide est largement utilisé pour les engrenages, les raccords et les roulements, dans l'industrie automobile pour les pièces sous-jacentes, et en tant que matériau pour les boîtiers d'outils électriques. Il est également utilisé dans la fabrication d'une grande variété de fils, de cordes, de filaments, de filets et de cordons de pneus, ainsi que de bonneterie et de vêtements tricotés.

Il existe une grande variété et nuances de types de polyamides disponibles industriellement, connus par les acronymes 'PAx.x'.

Les principaux points forts du polyamide sont ses très bonnes propriétés mécaniques (traction, fatigue, choc, résistance à l'abrasion), ainsi qu'une bonne résistance aux carburants et aux huiles. Il est par contre sensible à l'humidité de l'air ambiant, et présente une résistance aux UV assez limitée. Malgré tout, les polyamides présentent un excellent équilibre coût/performance.

Les polyamides sont actuellement très peu recyclés, pour des raisons liées à la chimie des polymères (le nylon est plus difficile à recycler que le polyester).

### POLYOLEFINES

Les polyéthylènes, de la famille des polyoléfines, font partie des plastiques dit « de grande diffusion », avec des consommations très importantes. Ils sont l'un des plastiques qui se prête au recyclage, même s'il est souvent destiné à des applications à faible valeur ajoutée. Il se subdivise en nombreuses sous catégories, chacune possédant des caractéristiques spécifiques (PEHD, PEBD, PEBDL, UWMWPE...).

Les fibres de polyéthylène haute ténacité (couramment appelé « Ultra-High Molecular Weight Polyethylene », ou UHMWPE) présentent les avantages de la légèreté (densité de 0,95 à comparer à celle de l'aramide de 1,44) et d'une haute capacité de conversion de l'énergie cinétique en énergie thermique. Elles sont de plus en plus utilisées dans les gilets pare-balles et autres applications balistiques, en concurrence avec le Kevlar, afin d'en alléger le poids.

Les fibres en polyéthylène de la gamme Dyneema^{™} (du fabricant DSM) et Spectra^{™} (du fabricant Honeywell) se caractérisent par leur très forte résistance, et ceci pour un poids minimal. En effet, à poids égal, une telle fibre est jusqu'à 15 fois plus résistante que l'acier fin et 40 % plus solide qu'une fibre d'aramide. En outre, elle est plus légère que l'eau, extrêmement durable et elle résiste aux moisissures, aux rayons UV et aux produits chimiques.

Du coté des inconvénients, il faut noter une mauvaise résistance à la température (fluage dès 90°C ; l'aramide ne se dégrade qu'à 400°C), ainsi que de mauvaises propriétés d'adhérence qui rendent délicates les applications composites (traitement de surface nécessaire).

### TITANE

Le titane est un métal léger et résistant, considéré comme « noble ». Il présente des propriétés industrielles intéressantes telles que sa résistance à la corrosion, à l'érosion et au feu. Il est ductile et biocompatible et présente également des propriétés mécaniques qui permettent de façonner des pièces fines et légères.

Du fait de ses nombreuses qualités, il est utilisé dans beaucoup de domaines à forte valeur ajoutée : médical, aéronautique, pétrochimie et nouveaux sports mécaniques et de loisirs, où l'allègement devient un gage de performance. Le titane est un métal qui réunit des qualités mécaniques fort intéressantes et une excellente tenue dans les milieux corrosifs, ce qui évite des traitements de surface et rend le titane « écologique ». De plus, sa densité est 2 fois plus faible que celle des aciers.

Le titane est principalement utilisé sous forme d'alliages dans l'aéronautique et pour de nombreuses applications industrielles (énergie, chimie, etc.). Etant donné la part importante de chutes de production, le recyclage des copeaux est bien organisé pour récupérer un maximum de matière. Il n'est pas rare de voir que les copeaux d'usinage peuvent représenter jusqu'à 90% de la consommation de titane pour la réalisation d'une pièce. Ces copeaux sont utilisés comme co-produits, comme dans le cas des fibres structurelles des cordages.

### CARBONE

Les fibres de carbone sont issues du pétrole et présentent des propriétés extrêmement intéressantes : une raideur et stabilité mécanique à toute épreuve, un poids ultra léger et une insensibilité aux rayons UV. A condition qu'elle ne soit pas exposée aux chocs, la durée de vie de la fibre de carbone est virtuellement illimitée.

On retrouve aujourd'hui les fibres de carbone dans bon nombre d'applications techniques de pointes, où la résistance mécanique, alliée à une densité très faible sont de bons atouts : équipements sportifs, automobile, aéronautique, robotique, équipements militaires, hélices d'hélicoptère, éoliennes, drones.

L'une des technologies les plus adaptées pour le recyclage des fibres de carbone est la pyrolyse. Le matériau est soumis à une haute température (entre 400°C et 700°C), afin de conduire à une dégradation de la résine et une séparation des constituants. On obtient ainsi, selon les conditions, des résidus solides ou gazeux, qui peuvent être utilisés comme combustibles (valorisation énergétique, voir ci dessous). Les fibres peuvent être récupérées à l'issue du procédé pour être réintroduites dans des plastique ou composites. Le principal avantage de cette technique est la conservation des propriétés mécaniques des fibres de carbone recyclées.

### PROCESSUS

Après la collecte, le processus de recyclage commence par une étape d'identification (notée S0) des cordages. Pour les cordages de raquettes de tennis en particulier, l'étape d'identification consiste à pratiquer une coupe transversale (par ciseau, cutter ou autre outil tranchant) suite à quoi on prend une photo avec grossissement, par exemple au moyen d'un smartphone^{®} ou d'un appareil photo numérique.

L'étape d'identification par coupe et cliché comprend une analyse des groupes de filaments, pour en déduire une classification parmi au moins les types suivants : multi filaments simple, multi-filaments multi-cœur, multi-cœur multi-gaine, multi-cœur mono-gaine. On peut consulter ensuite sur une web-page une base de référence de sections et déterminer la section qui se rapproche le plus du cliché obtenu juste avant. Ce processus peut avantageusement être supporté par une application smartphone^{®}.

Selon une autre possibilité, l'étape d'identification consiste à relever une référence inscrite sur le fil de cordage lui-même. On consulte ensuite sur une page internet la fiche d'identification du produit et on y trouve le type de structure filamenteuse et enrobage.

Selon une autre possibilité, l'étape d'identification consiste à relever une marque représentative du fabricant du fil de cordage.

Selon encore une autre possibilité, la couleur ou les couleurs présentes sur la gaine extérieure du fil de cordage peuvent servir à déterminer le type de cordage, ce qui forme une autre méthode pour l'étape d'identification S0.

Selon encore une autre possibilité, on relève à la fois une marque représentative du fabricant et une couleur représentative du type de fil de cordage pour conclure à une identification du type de fils de cordage à recycler (étape S0).

Après identification, le processus prévoit de sélectionner une ou plusieurs étapes ci-dessous afin de séparer le fil de cordage en composants ou en éléments unitaires de petite taille.

De préférence, l'étape de collecte des cordages usagés confortera l'usage de bacs de récupération, notamment de bacs sélectifs, chacun prévu pour recevoir un type de cordage particulier.

### S1 - étape de séparation mécanique

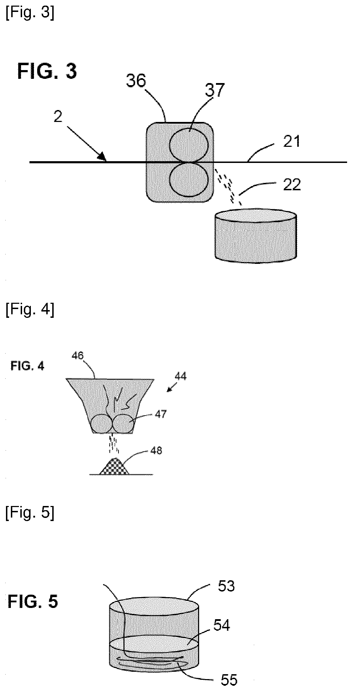

La figure 3 illustre une étape pelage et/ou une étape de broyage du fil de cordage. Lorsque la longueur des fils de cordage à traiter le permet, le fil de cordage peut être passé dans une machine similaire à une extrudeuse 36 représentée à la figure 3.

Dans cette machine 36, des râpes montés sur des rouleaux 37 viennent prendre en sandwich et grâce au mouvement rotatif des rouleaux, les râpes viennent peler le matériau d'enrobage au moins pour sa partie périphérique sur la section du fil de cordage. Le cœur 21 du fil de cordage est sous tension et ressort de la machine sans l'enrobage périphérique 22 qui est récupéré dans un bac à la sortie de la machine de pelage 36. La disposition est horizontale dans l'exemple illustré. Il peut y avoir plusieurs râpes en série (plusieurs passages de pelage).

Quant à l'étape de broyage, cette dernière permet de découper le fil de cordage en petit morceaux qui peuvent être traités ensuite par une étape de séparation chimique, thermique ou biologique. Pour cela comme illustré à la figure 4, une broyeuse 44 comprend une trémie 46 dans laquelle on déverse de morceaux de fil de cordage. Deux (ou plus) rouleaux contra-rotatif 47, grâce à une pluralité de dents de surface, déchiquètent les fils de cordages. A la sortie côté bas, des éléments unitaires 48 de petite taille s'amassent dans un récipient.

### S2 - étape de séparation chimique

Dans ce cas de figure, on utilise un solvant pour séparer de manière complète et fiable les fibres techniques du matériau d'enrobage. On peut utiliser du trichloréthylène, du trichloréthane, du dichlorométhane, du tétrachloroéthane, de l'acétone, etc ....

La figure 5 illustre un équipement de dissolution comprenant un bac 53 empli d'une solution avec le solvant précité 54. Des fils de cordages 55 y sont plongés, sans contrainte particulière sur leur longueur (brins courts, brins longs).

Au bout d'un temps prédéterminé, l'action de la solution solvante est considérée suffisante et on passe eu tamis la solution résultante, les fibres sont retenues par le tamis et le matériau d'enrobage dissous dans solution solvante passe au travers.

### S3 - étape de séparation thermique

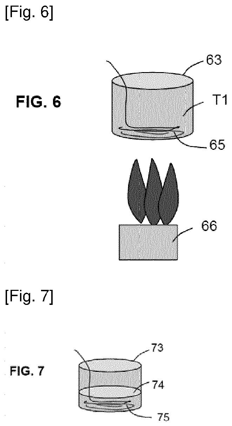

La figure 6 illustre un équipement une étape de vapo-thermolyse, avec un réchauffement du fil de cordage au-delà de 150°. On a représenté pour le principe un équipement à poste fixe, mais il est aussi prévu une solution avec déplacement progressif.

En fonction des points de fusions des différents composants, on sépare progressivement les constituants en augmentant la température. On récupére d'abord les constituants qui ont le point de fusion de fusion le plus bas et en dernier les composants qui ont le point de fusion de fusion le plus haut.

Sur l'exemple représenté, on place des fils de cordages 65, sans contrainte particulière sur leur longueur (brins courts, brins longs), dans un four 63 puis on chauffe à une première température prédéterminée T1 par exemple 160°C afin de faire fondre un des constituants du fil de cordage. Le chauffage 66 peut être fait par différents moyens, un brûleur, des rampes de lampes infra rouges, un four à induction, etc ...

### On extrait alors la partie fondue et on sépare le restant.

Bien entendu, il est possible de répéter l'opération décrite ci-dessus avec une deuxième température prédéterminée T2 par exemple 220°C afin de faire fondre un autre constituant du fil de cordage que l'on sépare alors comme partie fondue.

Selon un exemple, dans la logique des températures croissantes, on peut d'abord séparer les polyoléfines, ensuite les polyesters, ensuite les polyuréthanes, ensuite les polyamides, etc... Les fibres de carbone et de titane sont les derniers composants à rester.

### S4 - étape de séparation biologique

L'étape de séparation biologique comprend la mise en contact prolongée des fils de cordage avec des agents biologiques actifs comprenant des micro-algues et/ou des enzymes.

Ainsi, certains composants d'intérêt des fils de cordage sont dégradés par lesdits agents biologiques actifs. On remarque que, même si le temps nécessaire est plus long, cette solution est la plus économique en énergie pour parvenir à la séparation des composants.

Par ailleurs, on utilise également une telle étape de séparation biologique pour traiter par recyclage les cordages en boyau naturel. Les agents biologiques actifs comprenant des micro-algues et/ou des enzymes dégradent les cordages en boyau naturel en composants chimiques élémentaires qui n'ont alors plus aucun impact négatif sur l'environnement.

La figure 7 illustre un équipement de dissolution comprenant un bac 73 empli d'une solution avec des enzymes gloutons 74 et/ou des micro-algues. Des fils de cordages 75 y sont plongés, sans contrainte particulière sur leur longueur (brins courts, brins longs).

Au bout d'un temps prédéterminé, l'action des enzymes gloutons et/ou des micro-algues est considérée suffisante et on passe eu tamis la solution résultante, les fibres sont retenues par le tamis et le matériau d'enrobage dissous dans solution passe au travers.

### Valorisation des constituants séparés

En outre, on prévoit une étape de valorisation des produits/composants issus d'une des étapes /S1/ à /S4/.

L'étape de valorisation comprend par exemple l'incorporation des composants dans de nouveaux cordages, et/ou dans des vêtements techniques à rembourrage, et/ou dans des vêtements techniques de protection, et/ou dans des vêtement techniques ignifuges.

L'étape de valorisation peut comprendre une valorisation énergétique, selon laquelle on brûle un ou plusieurs résidus de l'étape de séparation susmentionnée.

### Autres considérations

Il faut noter que les différentes étapes et solutions pour les étapes d'identification, séparation et valorisation, sont applicables, mutatis mutandis, à d'autres types de cordages que les cordages de tennis, en particulier les cordages techniques utilisés pour la voile, la varappe, l'alpinisme ou encore d'autres sports, ainsi que les cordages d'instruments de musique à cordes.

## Revendications

1. Procédé pour recycler des cordages composites, le procédé comprenant:
- une étape d'identification (S0) du type de cordage,
et au moins une étape de séparation de composants comprenant l'une ou l'autre des étapes suivantes /S1/,/S2/,/S3/,/S4/ telles que définies ci-après:
/S1 / - une étape de séparation mécanique,
/S2/ - une étape de séparation chimique,
/S3/ - une étape de séparation thermique,
/S4/ - une étape de séparation biologique,
procédé dans lequel les cordages à recycler sont des cordages techniques utilisés pour des raquettes de tennis, pour la voile, la varappe, l'alpinisme ou encore d'autres sports, ou des cordages d'instruments de musique à cordes, le procédé étant **caractérisé en ce que** :
- l'étape d'identification (SO) pour les cordages consiste à pratiquer une coupe transversale, prendre une photo avec grossissement, et analyser les groupes de filaments, pour en déduire une classification parmi au moins les types suivants : multi filaments simple, multi-filaments multi-cœur, multi-cœur multi-gaines, multi-cœur mono-gaine.

2. Procédé selon la revendication 1, dans lequel les cordages de raquettes de tennis comprennent un assemblage de fibres de structure (3) avec un matériau d'enrobage (4), dans lequel les fibres de structure comprend des fibres d'aramide et/ou de polyester et/ou de polyamide et/ou de polyoléfine et/ou de polyéthylène, et dans lequel le matériau d'enrobage comprend du polyuréthane et/ou un élastomère.

3. Procédé selon la revendication 2, dans lequel selon l'étape de séparation mécanique (/S1/) des fibres et de l'enrobage, il est prévu une étape de pelage et/ou une étape de broyage du fil de cordage.

4. Procédé selon la revendication 2 ou 3, dans lequel l'étape de séparation chimique (/S2/) comprend la dissolution du matériau d'enrobage des fibres dans un solvant.

5. Procédé selon l'une des revendications 2 à 4, dans lequel l'étape de séparation thermique (/S3/) comprend une étape de vapo-thermolyse, avec un réchauffement du fil de cordage au-delà de 150°C.

6. Procédé selon l'une des revendications 2 à 5, dans lequel l'étape de séparation biologique (/S4/) comprend la mise en contact prolongée des fils de cordage avec des agents biologiques actifs comprenant des micro-algues et/ou des enzymes, de sorte que certains composants d'intérêt des fils de cordage sont dégradés par les agents biologiques actifs.

7. Procédé selon l'une des revendications 1 à 6, dans lequel il est prévu une étape de valorisation des produits/composants issus d'une des étapes de séparation /S1/ à /S4/.

8. Procédé selon la revendication 7, dans lequel l'étape de valorisation comprend l'incorporation des composants dans de nouveaux cordages, et/ou dans des vêtements techniques à rembourrage, et/ou dans des vêtements techniques de protection, et/ou dans des vêtement techniques ignifuges.

9. Procédé selon la revendication 7, dans laquelle l'étape de valorisation comprend une valorisation énergétique, selon laquelle on brûle un ou plusieurs résidus de l'étape de séparation susmentionnée.

## Patentansprüche

1. Verfahren zum Recycling von Verbundsaiten, umfassend:
- einen Identifikationsschritt (S0) der bespannungsart,
und mindestens einen Komponententrennungsschritt, der einen der folgenden schritte/S1/,/S2/,/S3/,/S4/ umfasst, wie unten definiert:
/S1/ - einen mechanischen trennschritt,
/S2/ - einen chemischen trennschritt,
/S3/ - einen thermischen trennschritt,
/S4/ - ein biologischer trennschritt,
Die Erfindung betrifft außerdem ein Verfahren, bei dem es sich bei den zu recycelnden Saiten um Tennisschlägersaiten handeln kann und bei dem:
- Der Identifizierungsschritt (S0) besteht darin, einen querschnitt zu erstellen, ein foto mit vergrößerung aufzunehmen und die filamentgruppen zu analysieren, um daraus eine klassifizierung aus mindestens den folgenden typen abzuleiten: mehradrige mehradrige, mehradrige mehradrige, mehradrige mehradrige filamente

2. Verfahren nach anspruch 1, wobei die sehnenfäden eine anordnung von strukturfasern (3) umfassen, die mit einem vergussmaterial (4) einen bespannungsdraht (3) bilden, wobei die strukturfasern aramid- und/oder polyester- und/oder polyamid- und/oder polyolefin- und/oder polyethylenfasern enthalten können und wobei das vergussmaterial polyurethan und/oder ein elastomer umfasst.

3. Verfahren nach anspruch 2, wobei im Schritt der mechanischen trennung (/S1/) der fasern und der beschichtung ein schälschritt und/oder ein schritt des schleifens des bespannungsdrahtes vorgesehen ist.

4. Verfahren nach anspruch 2 oder 3, umfassend in einem chemischen trennschritt (/S2/) das auflösen des beschichtungsmaterials der fasern in einem lösungsmittel.

5. Verfahren nach einem der ansprüche 2 bis 4, wobei der thermische trennschritt (/S3/) einen dampfthermolyseschritt mit einer erwärmung des bespannungsdrahtes über 150°C umfasst.

7. Verfahren nach einem der Ansprüche 1 bis 6, wobei ein Schritt der Aufbereitung der Produkte/Komponenten aus einem der Trennschritte /S1/bis /S4/ vorgesehen ist.

8. Verfahren nach Anspruch 7, bei dem der Wiederherstellungsschritt die Einarbeitung der Komponenten in neue Saiten und/oder in polsternde technische Kleidung und/oder in schützende technische Kleidung und/oder in flammhemmende technische Kleidung umfasst.

9. Verfahren nach Anspruch 7, bei dem der rückgewinnungsschritt eine energieaufbereitung umfassen kann, wobei ein oder mehrere rückstände des vorgenannten trennschritts verbrannt werden können.

## Claims

1. Method for recycling composite strings, comprising :
- an identification step (S0) of the type of stringing,
and at least one component separation step comprising one or the other of the following steps/S1/,/S2/,/S3/,/S4/as defined below
:/S1/ - a mechanical separation step,
/S2/ - a chemical separation step,
/S3/ - a thermal separation step,
/S4/ - a biological separation step,
The invention also relates to a method in which the strings to be recycled can be tennis racket strings and in which :
- Identification step (S0) consists in making a cross-section, taking a photo with magnification, and analysing the groups of filaments, in order to deduce therefrom a classification from at least the following types : multi-core multi-core, multi-core multi-core, multi-core multi-core filaments.

2. Method according to claim 1, wherein said chordae comprises an assembly of structural fibers (3) forming a stringing wire (3) with a potting material (4), wherein the structural fibers can contain aramid and/or polyester and/or polyamide and/or polyolefin and/or polyethylene fibers, and wherein the potting material comprises polyurethane and/or an elastomer.

3. Method according to claim 2, wherein, in the step of mechanical separation (/S1/) of the fibres and of the coating, there is provided a peeling step and/or a step of grinding the stringing wire.

4. Method according to Claim 2 or 3, comprising, in a chemical separation step (/S2/), the dissolution of the coating material of the fibres in a solvent.

5. Method according to one of claims 2 to 4, wherein the thermal separation step (/S3/) comprises a vapor-thermolysis step, with a heating of the stringing wire beyond 150°C.

6. Method according to one of claims 2 to 5, wherein the biological separation step (/S4/) comprises prolonged contacting of the stringing wires with active biological agents comprising microalgae and/or enzymes, so that certain components of interest of the stringing wires are degraded by the active biological agents.

7. Method according to one of claims 1 to 6, wherein there is provided a step of upgrading the products/components from one of the separation steps/S1/to/S4/.

8. Method according to claim 7 in which the recovery step comprises the incorporation of the components into new string, and/or in cushioning technical clothing, and/or in protective technical clothing, and/or in flame-retardant technical clothing.

9. Method according to claim 7 in which the recovery step can comprise energy upgrading, wherein one or more residues of the aforementioned separation step can be burned.
